# EUROPEAN PATENT APPLICATION

(11) **EP 4 737 437 A1**
(43) Date of publication of application: **06.05.2026**
(21) Application number: 24830734.0
(22) Date of filing: 25.06.2024
(51) Int. Cl.: C07C 269/04

(54) **DPP1 INHIBITOR INTERMEDIATE, AND PREPARATION METHOD THEREFOR AND USE THEREOF IN MEDICINES**

(30) Priority: 27.06.2023 CN 202310763816
(71) Applicant: Haisco Pharmaceuticals Pte. Ltd., Singapore 079903 (SG)
(72) Inventor: FAN, Jiang, Chengdu, Sichuan 611130 (CN); ZHU, Fengfei, Chengdu, Sichuan 611130 (CN); DOU, Ying, Chengdu, Sichuan 611130 (CN)
(74) Representative: Bianchetti & Minoja with Trevisan & Cuonzo IPS SRL
(86) International application number: PCT/CN2024/101177
(87) International publication number: WO 2025/002093

(57) **Abstract**

The present disclosure relates to a compound as represented by formula (I) and a method for preparing an intermediate thereof. The method uses inexpensive starting materials and multi-step telescoping reactions, is simple and convenient in terms of post-treatment operation, achieves a high yield, is high in terms of the chemical and chiral purity of a product, and is suitable for large-scale industrial production.

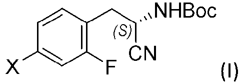

## Description

### TECHNICAL FIELD

The present disclosure relates to a method for preparing a compound as represented by formula (I) and an intermediate thereof. The method features low-cost reaction starting materials, multi-step telescoping reactions, straightforward workup procedures, high yield, and high chemical and chiral purity of the product, making it suitable for large-scale industrial production.

### BACKGROUND ART

Dipeptidyl peptidase 1 (DPP1), also known as cathepsin C, is a cysteinyl protease of the lysosomal papain family involved in intracellular protein degradation. During neutrophil maturation, DPP1 activates neutrophil serine proteases (NSPs), including neutrophil elastase (NE), proteinase 3 (Pr3), and cathepsin G (CatG), by cleaving the N-terminal dipeptide of a target protein. DPP1 has been implicated in a variety of inflammatory diseases, including Wegener's granulomatosis, rheumatoid arthritis, lung inflammation, virus infection, etc. Studies have shown that the inhibition of DPP1 can have a good therapeutic effect on highly inflammatory lung diseases caused by neutrophils, such as bronchiectasis, chronic obstructive pulmonary disease (COPD) and acute lung injury. Therefore, inhibiting the over-activation of NSPs by targeting DPP1 may have a potential therapeutic effect on bronchiectasis.

WO 2014140075A1 and WO 2016016242A1 recite a class of compounds having DPP1 activity, wherein the compound as represented by formula (C7) 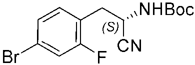 can be used as a key intermediate for the synthesis of this class of products.

WO 2014140075A1 discloses intermediate I-1.1, which is prepared via Route 1 using R8 and R9 as starting materials. This preparation method mainly has problems such as expensive starting materials, the requirement for ultra-low reaction temperature, low reaction conversion efficiency, the necessity for purification by column chromatography, and the difficulty in industrial scale-up production.

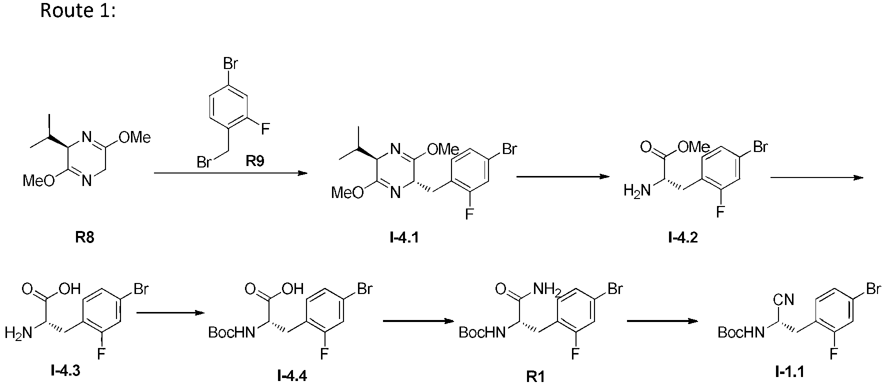

WO 2016016242A1 discloses another intermediate I-5.2.1, which is prepared via Route 2 using R14 as a starting material, as described below. This route mainly has problems such as the requirement for asymmetric reduction in the reaction, the high cost and susceptibility to deactivation of catalysts, the formation of dehalogenated by-products during the asymmetric reduction reaction, the necessity for purification by reversed-phase HPLC, high production costs, and the difficulty in scale-up production.

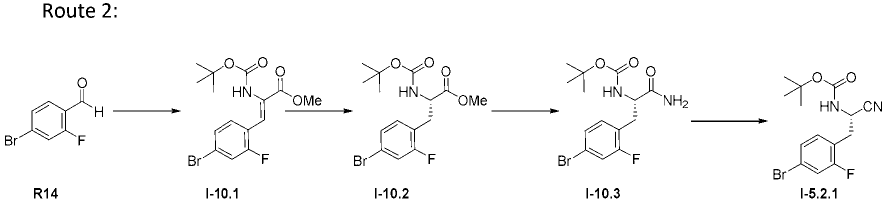

Therefore, it is necessary to develop a route for preparing compound (I) that features mild reaction conditions, simple operation, high reaction yield, high chemical and chiral purity of the product, straightforward workup, low cost, and the suitability for industrial production.

### SUMMARY OF THE INVENTION

The objective of the present disclosure is to provide a method for preparing a compound as represented by formula (I) and an intermediate thereof.

The method of the present disclosure features low-cost reaction starting materials, multi-step telescoping reactions, straightforward workup procedures, high yield, and high chemical and chiral purity of the product, making it suitable for large-scale industrial production.

The present disclosure provides a method for preparing a compound as represented by formula (I), wherein the compound as represented by formula (I) is prepared from a compound of formula (II), 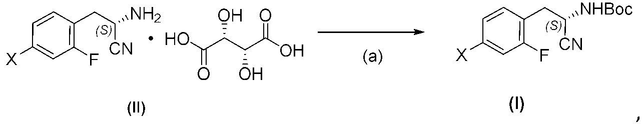 wherein X is a leaving group, such as F, Cl, Br, I or OTf.

The method for preparing the compound as represented by formula (I) provided by the present disclosure may further comprise the following: a compound of formula (II-1) is prepared from a compound of formula (III); a compound of formula (II) is prepared from the compound of formula (II-1); and then the compound as represented by formula (I) is prepared from the compound of formula (II): 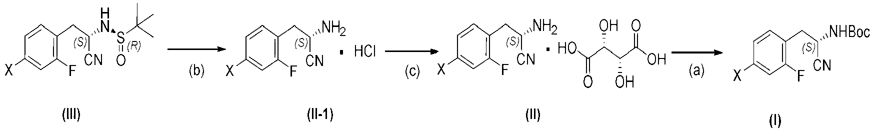 wherein X is a leaving group, such as F, Cl, Br, I or OTf.

The method for preparing the compound as represented by formula (I) provided by the present disclosure may further comprise the following: a compound of formula (III) is prepared from a compound of formula (IV); a compound of formula (II-1) is prepared from the compound of formula (III); a compound of formula (II) is prepared from the compound of formula (II-1); and then the compound as represented by formula (I) is prepared from the compound of formula (II): 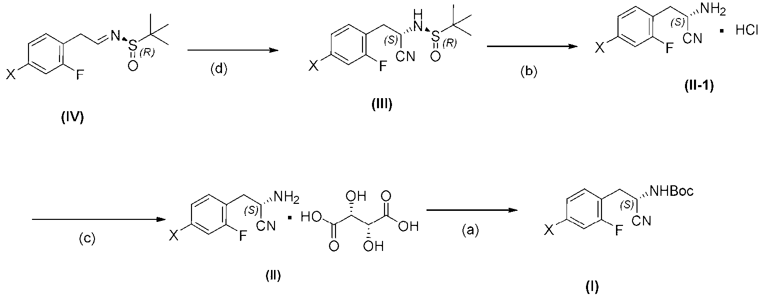 wherein X is a leaving group, such as F, Cl, Br, I or OTf.

The method for preparing the compound as represented by formula (I) provided by the present disclosure may further comprise the following: a compound of formula (VI) is prepared from a compound of formula (VII-1) and a compound of formula (VII-2); a compound of formula (V-1) is prepared from the compound of formula (VI); a compound of formula (IV) is prepared from the compound of formula (V-1) and a compound of formula (V-2); a compound of formula (III) is prepared from the compound of formula (IV); a compound of formula (II-1) is prepared from the compound of formula (III); a compound of formula (II) is prepared from the compound of formula (II-1); and then the compound as represented by formula (I) is prepared from the compound of formula (II): 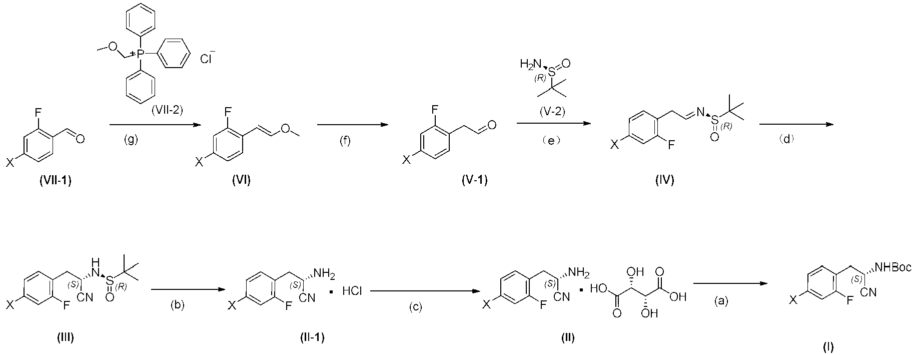 wherein X is a leaving group, such as F, Cl, Br, I or OTf.

The present disclosure provides a method for preparing a compound as represented by formula (II), wherein a compound of formula (II-1) is prepared from a compound of formula (III); and the compound of formula (II) is prepared from the compound of formula (II-1), 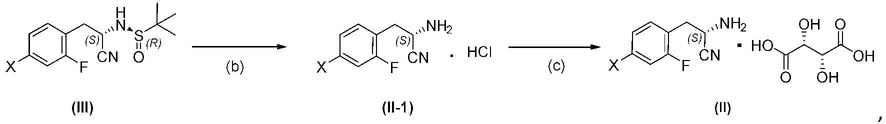 wherein X is a leaving group, such as F, Cl, Br, I or OTf.

The method for preparing the compound as represented by formula (II) provided by the present disclosure may further comprise the following: a compound of formula (III) is prepared from a compound of formula (IV); a compound of formula (II-1) is prepared from the compound of formula (III); and then the compound of formula (II) is prepared from the compound of formula (II-1): 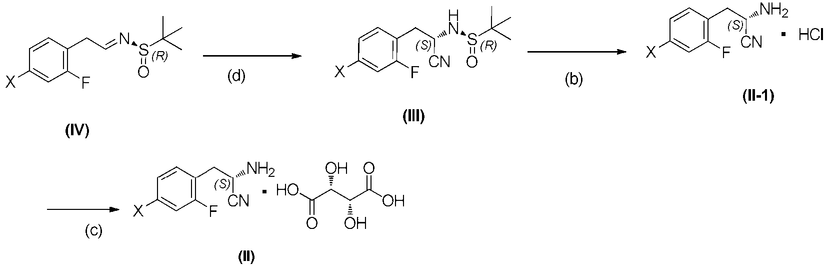 wherein X is a leaving group, such as F, Cl, Br, I or OTf.

The method for preparing the compound as represented by formula (II) provided by the present disclosure may further comprise the following: a compound of formula (VI) is prepared from a compound of formula (VII-1) and a compound of formula (VII-2); a compound of formula (V-1) is prepared from the compound of formula (VI); a compound of formula (IV) is prepared from the compound of formula (V-1) and a compound of formula (V-2); a compound of formula (III) is prepared from the compound of formula (IV); a compound of formula (II-1) is prepared from the compound of formula (III); and then the compound of formula (II) is prepared from the compound of formula (II-1): 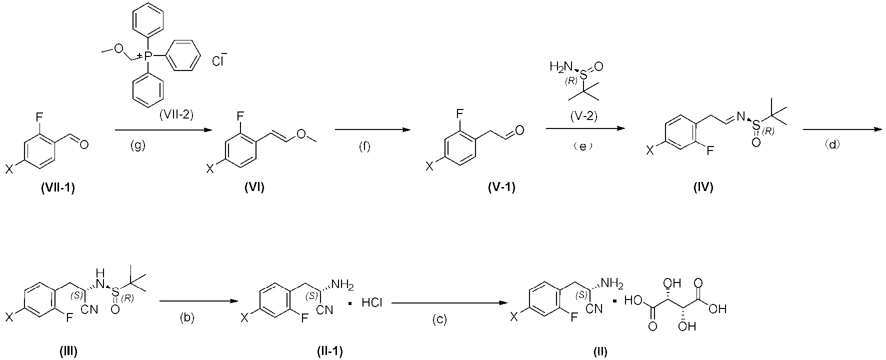 wherein X is a leaving group, such as F, Cl, Br, I or OTf.

The present disclosure provides a method for preparing a compound as represented by formula (III), wherein the compound of formula (III) is prepared from a compound of formula (IV): 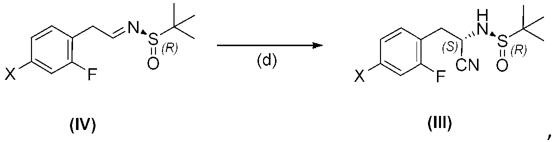 wherein X is a leaving group, such as F, Cl, Br, I or OTf.

The method for preparing the compound as represented by formula (III) provided by the present disclosure may further comprise the following: a compound of formula (VI) is prepared from a compound of formula (VII-1) and a compound of formula (VII-2); a compound of formula (V-1) is prepared from the compound of formula (VI); a compound of formula (IV) is prepared from the compound of formula (V-1) and a compound of formula (V-2); and the compound of formula (III) is prepared from the compound of formula (IV): 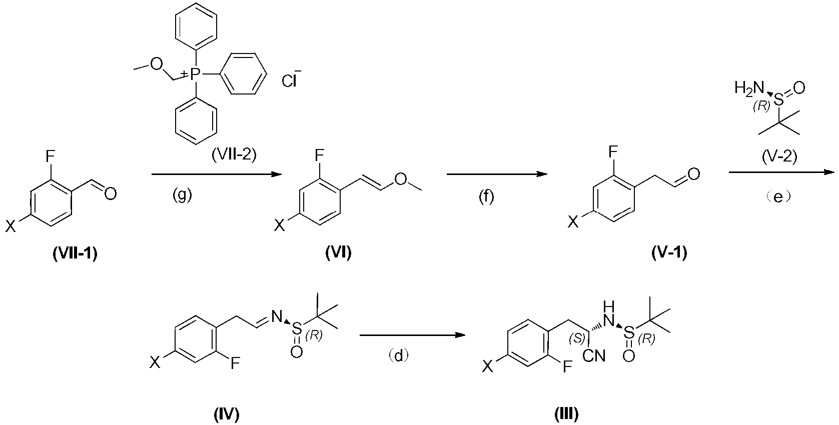 wherein X is a leaving group, such as F, Cl, Br, I or OTf.

The present disclosure provides a method for preparing a compound as represented by formula (IV), wherein a compound of formula (VI) is prepared from a compound of formula (VII-1) and a compound of formula (VII-2); a compound of formula (V-1) is prepared from the compound of formula (VI); and the compound of formula (IV) is prepared from the compound of formula (V-1) and a compound of formula (V-2), 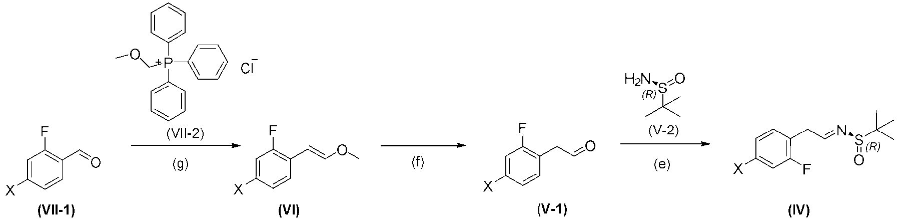 wherein X is a leaving group, such as F, Cl, Br, I or OTf.

In some embodiments of the method for preparing the compound of formula (I) involved in the present disclosure, the method comprises the following steps : 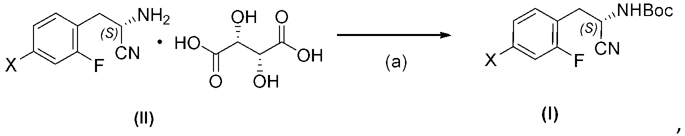
wherein X is a leaving group, such as F, Cl, Br, I or OTf;
a: the compound as represented by formula (I) is prepared from a compound of formula (II).

In some embodiments of the method for preparing the compound of formula (I) as described above involved in the present disclosure, the method may further comprise the following steps: 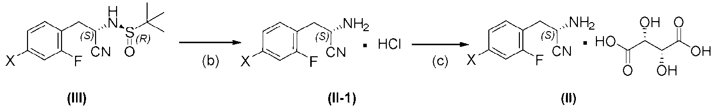
wherein X is a leaving group, such as F, Cl, Br, I or OTf;
b: a compound of formula (II-1) is prepared from a compound of formula (III);
c: a compound of formula (II) is prepared from the compound of formula (II-1).

In some embodiments of the methods for preparing the compound of formula (I) and the compound of formula (II) as described above involved in the present disclosure, the method further comprises the following steps: 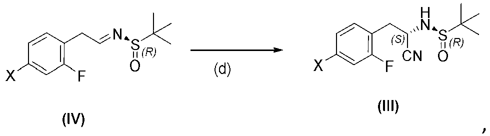
wherein X is a leaving group, such as F, Cl, Br, I or OTf;
d: a compound of formula (III) is prepared from a compound of formula (IV).

In some embodiments of the methods for preparing the compound of formula (I), the compound of formula (II) and the compound of formula (III) as described above involved in the present disclosure, the method may further comprise the following steps: 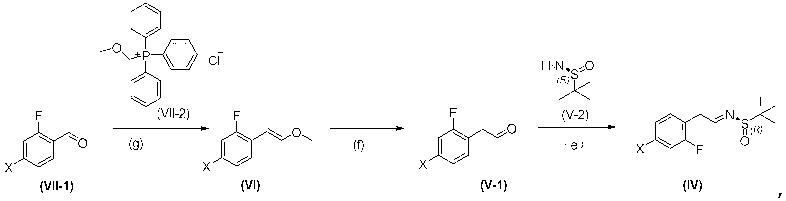
wherein X is a leaving group, such as F, Cl, Br, I or OTf.
g: a compound of formula (VI) is prepared from a compound of formula (VII-1) and a compound of formula (VII-2);
f: a compound of formula (V-1) is prepared from the compound of formula (VI);
e: a compound of formula (IV) is prepared from the compound of formula (V-1) and a compound of formula (V-2).

In some embodiments of the method for preparing the compound of formula (I) as described above involved in the present disclosure,
a: a compound as represented by formula (II) is reacted in the presence of an organic solvent and a base to obtain the compound as represented by formula (I), wherein the organic solvent is preferably one or more of dichloromethane, ethyl acetate, isopropyl acetate, toluene, dioxane, tetrahydrofuran, methyltetrahydrofuran, MTBE, (Boc)₂O or n-heptane, more preferably one or more of dichloromethane, MTBE, (Boc)₂O or n-heptane; and the base is preferably one or more of sodium carbonate, sodium bicarbonate, potassium carbonate, potassium bicarbonate, potassium phosphate, potassium hydroxide, lithium hydroxide, sodium hydroxide, triethylamine, DIPEA or DBU, further preferably sodium bicarbonate, sodium carbonate or sodium hydroxide, more preferably sodium carbonate.

In some embodiments of the method for preparing the compound of formula (I) as described above involved in the present disclosure, the ratio of the amount of the compound as represented by formula (II) to the organic solvent is 1 : 3-20, preferably 1 : 4-8.

In some embodiments of the method for preparing the compound of formula (I) as described above involved in the present disclosure, the reaction temperature is -10°C-60°C, preferably 0°C-30°C.

In some embodiments of the method for preparing the compound of formula (I) as described above involved in the present disclosure, the reaction time is 10-48 h, preferably 15-20 h.

In some embodiments of the method for preparing the compound of formula (I) as described above involved in the present disclosure, the drying time is 8-48 h, preferably 12-24 h.

In some embodiments of the method for preparing the compound of formula (I) as described above involved in the present disclosure, the addition ratio of the base is 1 : 0.5-3.0, preferably 1 : 1.0-1.5.

In some embodiments of the methods for preparing the compound of formula (I) and the compound of formula (II) as described above involved in the present disclosure,
b: a compound of formula (III) is reacted in the presence of an organic solvent and an acid to obtain a compound of formula (II-1), wherein the organic solvent is preferably one or more of dichloromethane, ethyl acetate, isopropyl acetate, toluene, dioxane, tetrahydrofuran, methyltetrahydrofuran, MTBE, methanol, ethanol or isopropanol, more preferably ethanol or MTBE; the acid is preferably one or more of HCl, hydrobromic acid, hydroiodic acid, sulfuric acid, phosphoric acid, methanesulfonic acid, p-toluenesulfonic acid or trifluoroacetic acid, more preferably HCl;
c: the compound of formula (II-1) is reacted in the presence of a chiral acid to obtain a compound of formula (II), wherein the chiral acid is preferably one or more of tartaric acid, malic acid, camphoric acid, camphorsulfonic acid, lactic acid, diacetone-L-gulonic acid, mandelic acid or phenoxypropionic acid, more preferably L(-)-tartaric acid.

In some embodiments of the methods for preparing the compound of formula (I) and the compound of formula (II) as described above involved in the present disclosure, the equivalent of L(-)-tartaric acid can be selected from 0.5-2.0 eq, preferably 0.6-1.0 eq.

In some embodiments of the methods for preparing the compound of formula (I) and the compound of formula (II) as described above involved in the present disclosure, the ratio of the amount of the compound as represented by formula (III) to the organic solvent is 1 : 2.0-10.0, preferably 1 : 3-5.

In some embodiments of the methods for preparing the compound of formula (I) and the compound of formula (II) as described above involved in the present disclosure, the reaction temperature is -10°C-60°C, preferably 0°C-30°C.

In some embodiments of the methods for preparing the compound of formula (I) and the compound of formula (II) as described above involved in the present disclosure, the reaction time is 3-12 h, preferably 4-6 h.

In some embodiments of the methods for preparing the compound of formula (I) and the compound of formula (II) as described above involved in the present disclosure, the addition ratio of the acid is 1 : 1.0-6.0, preferably 1 : 2.0-4.0.

In some embodiments of the methods for preparing the compound of formula (I), the compound of formula (II) and the compound of formula (III) as described above involved in the present disclosure,
d: a compound of formula (IV) is reacted in the presence of a solvent, a cyanating reagent and a base to obtain the compound of formula (III), wherein the solvent is preferably one or more of DCM, DMF, THF, n-heptane, ACN, toluene or DMSO, more preferably DCM or toluene; the cyanating reagent is preferably one or more of TMSCN, sodium cyanide, potassium cyanide or lithium cyanide, more preferably TMSCN; and the base is preferably one or more of CsF, KF, TBAF, Na₂CO₃, K₃PO₄ or Ti(OEt)₄, more preferably CsF or KF, even more preferably CsF.

In some embodiments of the methods for preparing the compound of formula (I), the compound of formula (II) and the compound of formula (III) as described above involved in the present disclosure, the ratio of the amount of the compound as represented by formula (IV) to the cyanating reagent is 1 : 1.0-3.0, preferably 1 : 1.3-1.7.

In some embodiments of the methods for preparing the compound of formula (I), the compound of formula (II) and the compound of formula (III) as described above involved in the present disclosure, the reaction temperature is -10°C-30°C, preferably 0°C-10°C.

In some embodiments of the methods for preparing the compound of formula (I), the compound of formula (II) and the compound of formula (III) as described above involved in the present disclosure, the reaction time is 10-24 h, preferably 12-16 h.

In some embodiments of the methods for preparing the compound of formula (I), the compound of formula (II) and the compound of formula (III) as described above involved in the present disclosure, the base is used in a ratio of 1: 0.05-2.0, preferably 1 : 0.1-0.3.

In some embodiments of the methods for preparing the compound of formula (I), the compound of formula (II), the compound of formula (III) and the compound of formula (IV) as described above involved in the present disclosure,
g: a compound of formula (VII-1) and a compound of formula (VII-2) are reacted in the presence of a base to obtain a compound of formula (VI), wherein the base is preferably one or more of LiHMDS, NaHMDS, KHMDS, LDA, BuLi or potassium tert-butoxide, more preferably LiHMDS, NaHMDS or KHMDS, even more preferably LiHMDS.

In some embodiments of the methods for preparing the compound of formula (I), the compound of formula (II), the compound of formula (III) and the compound of formula (IV) as described above involved in the present disclosure, the equivalent of LiHMDS is 1.3 eq.

In some embodiments of the methods for preparing the compound of formula (I), the compound of formula (II), the compound of formula (III) and the compound of formula (IV) as described above involved in the present disclosure, the ratio of the amount of the compound as represented by formula (VII-1) to the base is 1 : 1.0-3.0, preferably 1 : 1.2-1.5.

In some embodiments of the methods for preparing the compound of formula (I), the compound of formula (II), the compound of formula (III) and the compound of formula (IV) as described above involved in the present disclosure, the reaction temperature is -10°C-30°C, preferably 0°C-10°C.

In some embodiments of the methods for preparing the compound of formula (I), the compound of formula (II), the compound of formula (III) and the compound of formula (IV) as described above involved in the present disclosure, the reaction time is 1-10 h, preferably 2-5 h.

In some embodiments of the methods for preparing the compound of formula (I), the compound of formula (II), the compound of formula (III) and the compound of formula (IV) as described above involved in the present disclosure,
f: a compound of formula (VI) is reacted in the presence of an acid and an organic solvent to obtain a compound of formula (V-1), wherein the acid is preferably one or more of TsOH, methanesulfonic acid, camphorsulfonic acid, hydrochloric acid, hydrobromic acid, hydroiodic acid, sulfuric acid, phosphoric acid, trifluoroacetic acid, oxalyl chloride or trimethylchlorosilane, more preferably TsOH;
the organic solvent is preferably one or more of dichloromethane, ethyl acetate, isopropyl acetate, toluene, dioxane, tetrahydrofuran, methyltetrahydrofuran, methyl tert-butyl ether, acetonitrile, methanol, ethanol or isopropanol, more preferably toluene.

In some embodiments of the methods for preparing the compound of formula (I), the compound of formula (II), the compound of formula (III) and the compound of formula (IV) as described above involved in the present disclosure, the equivalent of TsOH can be selected from 1.0 eq, 2.0 eq or 4.0 eq, preferably 2.0 eq.

In some embodiments of the methods for preparing the compound of formula (I), the compound of formula (II), the compound of formula (III) and the compound of formula (IV) as described above involved in the present disclosure, the ratio of the amount of the compound as represented by formula (VI) to the acid is 1 : 1.0-5.0, preferably 1 : 1.5-3.0.

In some embodiments of the methods for preparing the compound of formula (I), the compound of formula (II), the compound of formula (III) and the compound of formula (IV) as described above involved in the present disclosure, the reaction temperature is 40°C-100°C, preferably 60°C-80°C.

In some embodiments of the methods for preparing the compound of formula (I), the compound of formula (II), the compound of formula (III) and the compound of formula (IV) as described above involved in the present disclosure, the reaction time is 0.5-10 h, preferably 1-5 h.

In some embodiments of the methods for preparing the compound of formula (I), the compound of formula (II), the compound of formula (III) and the compound of formula (IV) as described above involved in the present disclosure, the organic solvent is used in a ratio of 1 : 5-15, preferably 1 : 7-12.

In some embodiments of the methods for preparing the compound of formula (I), the compound of formula (II), the compound of formula (III) and the compound of formula (IV) as described above involved in the present disclosure,
e: a compound of formula (V-1) and a compound of formula (V-2) are reacted in the presence of an organic solvent and a dehydration reagent to obtain a compound of formula (IV), wherein the organic solvent is selected from one or more of toluene, dichloromethane, ethyl acetate, isopropyl acetate, dioxane, tetrahydrofuran, methyltetrahydrofuran, acetic acid or acetonitrile, preferably toluene;
the dehydration reagent is preferably one or more of anhydrous CuSO₄, Zn(OAc)₂, Co(OAc)₂^{·}4H₂O, Na₂SO₄, MgSO₄, Ti(Oi-Pr)₄, DCC/DMAP, EDCI/DMAP or HOAc/molecular sieve, preferably anhydrous CuSO₄ or HOAc/molecular sieve.

In some embodiments of the methods for preparing the compound of formula (I), the compound of formula (II), the compound of formula (III) and the compound of formula (IV) as described above involved in the present disclosure, the equivalent of anhydrous copper sulphate is 3.0 eq.

In some embodiments of the methods for preparing the compound of formula (I), the compound of formula (II), the compound of formula (III) and the compound of formula (IV) as described above involved in the present disclosure, the amount of acetic acid is twice the volume of intermediate V-1 in an acetic acid/molecular sieve system.

In some embodiments of the methods for preparing the compound of formula (I), the compound of formula (II), the compound of formula (III) and the compound of formula (IV) as described above involved in the present disclosure, the ratio of the amount of the compound as represented by formula (V-1) to the dehydration reagent is 1 : 1.0-6.0, preferably 1 : 2.0-4.0.

In some embodiments of the methods for preparing the compound of formula (I), the compound of formula (II), the compound of formula (III) and the compound of formula (IV) as described above involved in the present disclosure, the reaction temperature is 10°C-80°C, preferably 30°C-50°C.

In some embodiments of the methods for preparing the compound of formula (I), the compound of formula (II), the compound of formula (III) and the compound of formula (IV) as described above involved in the present disclosure, the reaction time is 6-24 h, preferably 8-16 h.

In some embodiments of the methods for preparing the compound of formula (I), the compound of formula (II), the compound of formula (III) and the compound of formula (IV) as described above involved in the present disclosure, the organic solvent is used in a ratio of 1 : 5-20, preferably 1 : 8-16.

The present disclosure further provides a method for preparing a compound as represented by formula (C7): 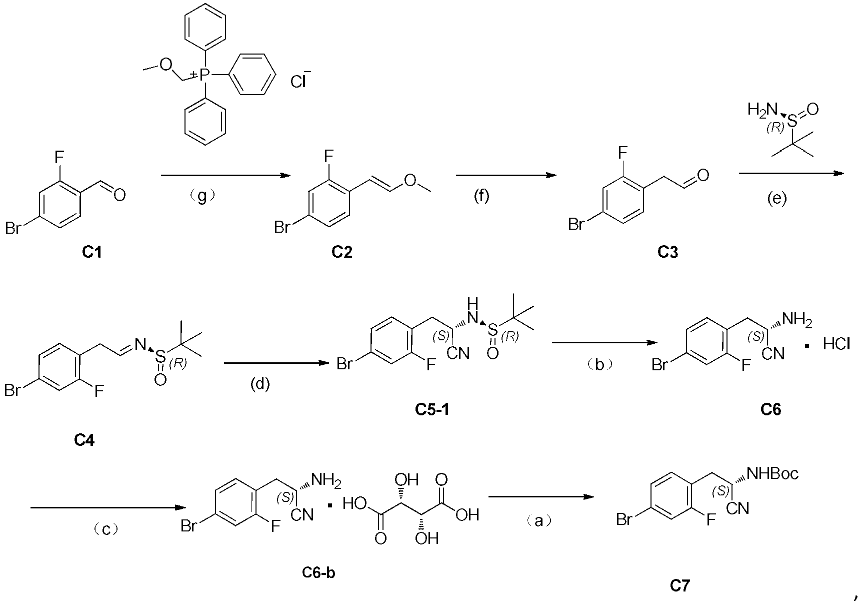 wherein
g: C1 is reacted in the presence of an organic solvent and a base to obtain C2;
f: C2 is reacted in the presence of an organic solvent and an acid to obtain C3;
e: C3 is reacted in the presence of an organic solvent and a dehydration reagent to obtain C4;
d: C4 is reacted in the presence of a solvent, a base and a cyanating reagent to obtain C5-1;
b: C5-1 is reacted in the presence of an acid to obtain C6;
c: C6 is reacted in the presence of an organic solvent, a base and a chiral acid to obtain C6-b;
a: C6-b is reacted in the presence of an organic solvent and a base to obtain C7.

In some embodiments of the method for preparing the compound of formula (C7) as described above involved in the present disclosure,
g: the organic solvent is selected from one or more of tetrahydrofuran, dichloromethane, MTBE or n-heptane;
the base is selected from one or more of LiHMDS, NaHMDS or KHMDS, preferably LiHMDS;
f: the acid is selected from one or more of TsOH, methanesulfonic acid, camphorsulfonic acid, hydrochloric acid, hydrobromic acid, hydroiodic acid, sulfuric acid, phosphoric acid, trifluoroacetic acid, oxalyl chloride or trimethylchlorosilane, preferably TsOH;
the organic solvent is selected from one or more of dichloromethane, ethyl acetate, isopropyl acetate, toluene, dioxane, tetrahydrofuran, methyltetrahydrofuran, methyl tert-butyl ether, acetonitrile, methanol, ethanol or isopropanol, preferably toluene;
e: the organic solvent is selected from one or more of toluene, dichloromethane, ethyl acetate, isopropyl acetate, dioxane, tetrahydrofuran, methyltetrahydrofuran, acetic acid or acetonitrile, preferably toluene; and the dehydration reagent is selected from one or more of anhydrous CuSO₄, Zn(OAc)₂, Co(OAc)₂^{·}4H₂O, Na₂SO₄, MgSO₄, Ti(Oi-Pr)₄, DCC/DMAP, EDCI/DMAP or HOAc/molecular sieve, preferably anhydrous CuSO₄ or HOAc/molecular sieve;
d: the solvent is selected from one or more of DCM, DMF, THF, n-heptane, ACN, toluene or DMSO, preferably DCM or toluene; the cyanating reagent is selected from one or more of TMSCN, sodium cyanide, potassium cyanide or lithium cyanide, preferably TMSCN; and the base is selected from one or more of CsF, KF, TBAF, Na₂CO₃, K₃PO₄ or Ti(OEt)₄, preferably CsF or KF, even more preferably CsF;
b: the organic solvent is selected from one or more of dichloromethane, ethyl acetate, isopropyl acetate, toluene, dioxane, tetrahydrofuran, methyltetrahydrofuran, MTBE, methanol, ethanol or isopropanol, preferably ethanol or MTBE; and the acid is selected from one or more of HCl, hydrobromic acid, hydroiodic acid, sulfuric acid, phosphoric acid, methanesulfonic acid, p-toluenesulfonic acid or trifluoroacetic acid, preferably HCl;
c: the chiral acid solvent is selected from one or more of tartaric acid, malic acid, camphoric acid, camphorsulfonic acid, lactic acid, diacetone-L-gulonic acid, mandelic acid or phenoxypropionic acid, preferably L(-)-tartaric acid;
a: the organic solvent is selected from one or more of dichloromethane, ethyl acetate, isopropyl acetate, toluene, dioxane, tetrahydrofuran, methyltetrahydrofuran, MTBE, (Boc)₂O or n-heptane, more preferably one or more of dichloromethane, MTBE, (Boc)₂O or n-heptane; and the base is selected from one or more of sodium carbonate, sodium bicarbonate, potassium carbonate, potassium bicarbonate, potassium phosphate, potassium hydroxide, lithium hydroxide, sodium hydroxide, triethylamine, DIPEA or DBU, preferably sodium carbonate.

In some embodiments of the method for preparing the compound of formula (C7) as described above involved in the present disclosure,
g: the organic solvent is tetrahydrofuran, and the base is LiHMDS;
f: the acid is TsOH, and the organic solvent is toluene;
e: the dehydration reagent is selected from anhydrous CuSO₄ or HOAc/molecular sieve;
d: the cyanating reagent is TMSCN, and the base is CsF;
b: the organic solvent is selected from ethanol or MTBE, and the acid is HCl;
c: the chiral acid solvent is L(-)-tartaric acid;
a: the organic solvent is selected from one or more of dichloromethane, MTBE, (Boc)₂O or n-heptane; and the base is sodium carbonate.

In some embodiments of the methods for preparing the compound of formula (I), the compound of formula (II), the compound of formula (III) and the compound of formula (IV) involved in the present disclosure, the workup in step g comprises refining the crude compound of formula (VI).

In some embodiments of the methods for preparing the compound of formula (I), the compound of formula (II), the compound of formula (III) and the compound of formula (IV) involved in the present disclosure, the refinement of the crude product in the step g workup comprises the following steps:
1) adding n-heptane to the crude concentrate of the compound of formula (VI), and stirring and filtering the mixture;
2) adding magnesium chloride to the filtrate, and stirring and filtering the mixture;
3) washing the filtrate with EDTA-Na, performing phase separation, and concentrating the organic phase; and adding toluene to the concentrate for distillation.

In some embodiments of the methods for preparing the compound of formula (I), the compound of formula (II), the compound of formula (III) and the compound of formula (IV) involved in the present disclosure, the workup in step f comprises refining the crude compound of formula (V-1).

In some embodiments of the methods for preparing the compound of formula (I), the compound of formula (II), the compound of formula (III) and the compound of formula (IV) involved in the present disclosure, the refinement of the crude product in the step f workup comprises the following steps:
washing the mixture containing a compound of formula (V-1) with a NaHCO₃ solution, performing phase separation, and collecting the organic phase to obtain the compound of formula (V-1).

In some embodiments of the methods for preparing the compound of formula (I), the compound of formula (II), the compound of formula (III) and the compound of formula (IV) involved in the present disclosure, the workup in step e comprises refining the crude compound of formula (IV).

In some embodiments of the methods for preparing the compound of formula (I), the compound of formula (II), the compound of formula (III) and the compound of formula (IV) involved in the present disclosure, the refinement of the crude product in the step f workup comprises the following steps:
washing the mixture containing a compound of formula (IV) with a NaHCO₃ solution, and performing phase separation to obtain the compound of formula (IV).

In some embodiments of the methods for preparing the compound of formula (I), the compound of formula (II) and the compound of formula (III) involved in the present disclosure, the workup in step d comprises refining the crude compound of formula (III).

In some embodiments of the methods for preparing the compound of formula (I), the compound of formula (II) and the compound of formula (III) involved in the present disclosure, the refinement of the crude product in the step d workup comprises the following steps:
1) adding a sodium carbonate solution to the compound of formula (III), and performing phase separation to obtain an organic phase;
2) adding a mixed solution of sodium carbonate and sodium chloride and a sodium chloride solution to the organic phase successively, performing phase separation, and collecting the organic phase to obtain the compound of formula (III).

In some embodiments of the methods for preparing the compound of formula (I) and the compound of formula (II) involved in the present disclosure, step b comprises the reaction and the workup.

In some embodiments of the methods for preparing the compound of formula (I) and the compound of formula (II) involved in the present disclosure, the reaction in step b comprises the following steps:
adding hydrochloric acid/ethanol to a compound of formula (III), controlling the temperature, and stirring the mixture for reaction.

In some embodiments of the methods for preparing the compound of formula (I) and the compound of formula (II) involved in the present disclosure, the refinement of the crude product in the step b workup comprises the following steps:
stirring and filtering the mixture, washing the filter cake, and collecting the filter cake to obtain a compound of formula (II-1).

In some embodiments of the methods for preparing the compound of formula (I) and the compound of formula (II) involved in the present disclosure, step c comprises the reaction and the workup.

In some embodiments of the methods for preparing the compound of formula (I) and the compound of formula (II) involved in the present disclosure, the reaction in step c comprises the following steps:
1) reacting a compound of formula (II-1) in the presence of dichloromethane and sodium carbonate;
2) performing phase separation, extracting the aqueous phase with dichloromethane, combining the organic phases, and performing concentration;
3) adding MeOH and L(-)-tartaric acid in methanol to the concentrate, and stirring the mixture for reaction.

In some embodiments of the methods for preparing the compound of formula (I) and the compound of formula (II) involved in the present disclosure, the workup in step c comprises the following steps:
filtering, washing the filter cake with MTBE, and collecting the filter cake to obtain a compound of formula (II-1).

In some embodiments of the method for preparing the compound of formula (I) involved in the present disclosure, the workup in step a comprises refining the crude compound of formula (I).

In some embodiments of the method for preparing the compound of formula (I) involved in the present disclosure, the refinement of the crude product in the step a workup comprises the following steps:
1) adding n-heptane to the compound of formula (I), stirring and heating the mixture, and performing concentration;
2) adding n-heptane, stirring and filtering the mixture, and washing the filter cake with a mixed solution of MTBE and n-heptane to obtain a wet product of the compound of formula (I);
3) controlling the temperature, and drying the wet product to obtain the compound of formula (I).

The method for preparing the compound as represented by formula (I) provided by the present disclosure has advantages such as low-cost reaction starting materials, multi-step telescoping reactions, straightforward workup procedures, high yield, high chemical and chiral purity of the product, and the suitability for large-scale industrial production.

Unless stated to the contrary, the terms used in the description and claims of the present application have the following meanings.

During the reaction of the present disclosure, the reaction process is tracked by HPLC, HNMR or thin layer chromatography so as to judge whether the reaction is completed.

In the present disclosure, the internal temperature indicates the temperature of the reaction system.

### DETAILED DESCRIPTION OF EMBODIMENTS

The technical solutions of the present disclosure will be described in detail by the following examples, but the scope of protection of the present disclosure includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is determined with NMR instruments (Bruker Avance III 400 and Bruker Avance 300); the solvent for determination is deuterated dimethylsulfoxide (DMSO-d₆), deuterated chloroform (CDCl₃) or deuterated methanol (CD₃OD); and the internal standard is tetramethylsilane (TMS).

MS is determined with Agilent 6120B (ESI) and Agilent 6120B (APCI).

HPLC is determined with Agilent 1260DAD high-pressure liquid chromatograph (Zorbax SB-C18 100 × 4.6 mm).

Yantai Huanghai HSGF₂₅₄ or Qingdao GF₂₅₄ silica gel plate is used as a thin layer chromatography silica plate; and the silica gel plate for the thin layer chromatography (TLC) is of the specification of 0.15 mm-0.20 mm, and the specification when separating and purifying a product by thin layer chromatography is 0.4 mm-0.5 mm.

Yantai Huanghai silica gel of 200-300 mesh is generally used as a carrier in column chromatography.

The known starting materials of the present disclosure can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.

The ratio shown in the silica gel column chromatography of the present disclosure is volume ratio.

The term "equivalent" in the present disclosure is a molar ratio.

The term "the ratio of the amount" is the ratio of the weight of the material (kg) to the volume of the solvent (L).
LDA: Lithium diisopropylamide
BuLi: n-Butyllithium
LiHMDS: Lithium bis(trimethylsilyl)amide
DMF: N,N-Dimethylformamide
THF: Tetrahydrofuran
DCM: Dichloromethane
TsOH: p-Toluenesulfonic acid
CuSO₄: Copper sulphate
Zn(OAc)₂: Zinc acetate
Co(OAc)₂^{·}4H₂O: Cobalt acetate hydrate
Na₂SO₄: Sodium sulfate
MgSO₄: Magnesium sulfate
Ti(Oi-Pr)₄: Titanium tetraisopropoxide
DCC/ DMAP: N,N'-Dicyclohexylcarbodiimide/4-dimethylaminopyridine
EDCI /DMAP: 1-(3-Dimethylaminopropyl)-3-ethylcarbodiimide hydrochloride/4-dimethylaminopyridine
HOAc/ molecular sieve: Acetic acid/molecular sieve system
CsF: Cesium fluoride
KF: Potassium fluoride
NH₄F: Ammonium fluoride
TBAF: Tetrabutylammonium fluoride
Na₂CO₃: Sodium carbonate solution
NaHCO₃: Sodium bicarbonate solution
NaOH: Sodium hydroxide
K₃PO₄: Potassium phosphate
Ti(OEt)₄: Isopropyl titanate
n-Heptane
ACN: Acetonitrile
Toluene
DMSO: Dimethylsulfoxide
TMSCN: Trimethylsilyl cyanide
Tartaric acid
MTBE: Methyl tert-butyl ether
DIPEA: N,N-Diisopropylethylamine
DBU: 1,8-Diazabicyclo[5.4.0]undec-7-ene
(Boc)₂O: Di-tert-butyl dicarbonate
OTF: Trifluoromethanesulfonate

### Example

### tert-butyl (S)-(2-(4-bromo-2-fluorophenyl)-1-cyanoethyl)carbamate (C7)

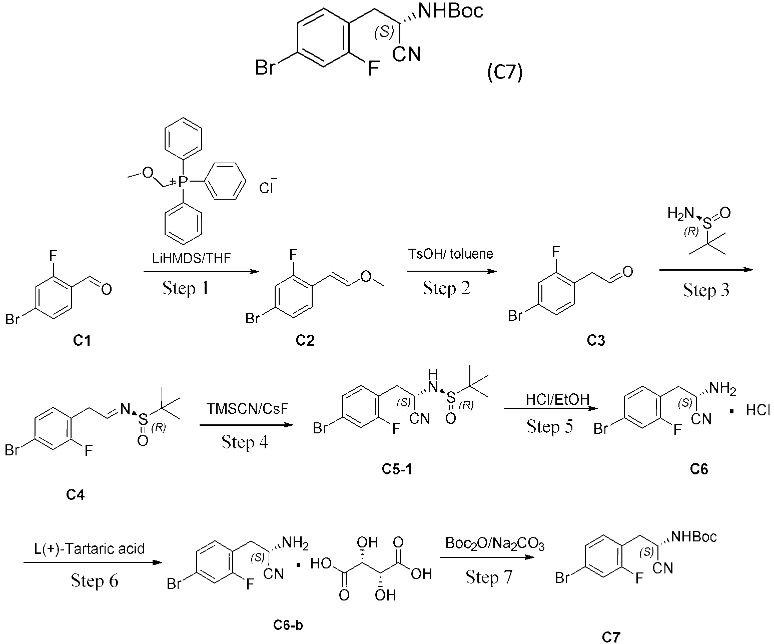

### Step 1: 4-bromo-2-fluoro-1-(2-methoxyvinyl)benzene (C2)

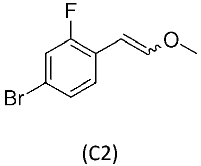

**Reaction:** To a reaction kettle were added THF (8V) and LiHMDS (1.34 eq). After the addition was completed, the mixture was stirred and cooled to 0°C ± 5°C under nitrogen atmosphere. (Methoxymethyl)triphenylphosphonium chloride (1.3 eq) was added in portions, and the mixture was stirred for about 2 h with the temperature maintained at 0°C ± 5°C. Then, a solution of C1 (53.0 kg, 1.0 eq) in THF (2V) was added dropwise with the temperature controlled at 0°C ± 5°C, and the mixture was reacted for about 2 hours with the temperature maintained at 0°C ± 5°C. The reaction mixture was sampled and monitored. When the in-process control limit of **C1/C1** + **C2 ≤ 2.0%** was met, the reaction was stopped.

**Workup:** Water (5V) was added to the reaction system to quench the reaction. The aqueous phase was extracted with MTBE (5V), the mixture was stirred and subjected to phase separation, and the organic phase was concentrated and subjected to rotary evaporation. To the concentrate was added n-heptane (10V), and the mixture was stirred for about 1 h and filtered. To the filtrate was added magnesium chloride (1.5 eq), and the mixture was stirred for about 16 h at 60°C ± 5°C and filtered. The filtrate was washed with 5% EDTA-Na (5V), and the phases were separated. The organic phase was concentrated and subjected to rotary evaporation, and the residue was subjected to azeotropic distillation with toluene (5V), to obtain compound C2 (48.3 kg, yield: 80.0%).

¹H NMR (300 MHz, CDCl₃) δ 7.84 (t, 1H), 7.17 - 7.04 (m, 1H), 6.18 (d, 1H), 5.33 (d, 1H), 3.74 (d, 2H).

GCMS m/z =230.00

### Step 2: 2-(4-bromo-2-fluorophenyl)acetaldehyde (C3)

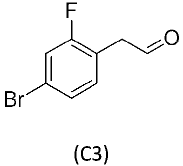

**Reaction:** To a reaction kettle were added C2 (53.0 kg, 1.0 eq), toluene (10V) and water (1V) under stirring. After the addition was completed, the mixture was purged three times with nitrogen and warmed to 70°C ± 5°C. p-Toluenesulfonic acid monohydrate (3.0 eq) was added, and the mixture was reacted for about 1 hour with the temperature controlled at 70°C ± 5°C. The reaction mixture was sampled and monitored. When the in-process control limit of C2/C2 + C3 **≤** 5.0% was met, the reaction was stopped.

**Workup:** After the reaction was completed, the phases were separated, and the aqueous phase was extracted twice with toluene (2V). The organic phases were combined and washed once with a 5% NaHCO₃ solution (4V). The phases were separated, and the organic phase was collected to obtain a solution of C3 in toluene (total weight: 755.9 kg, content: 4.90%, yield: 74.1%), which was directly used in the next reaction.

GCMS: 215.90.

### Step 3: (R,E)-N-(2-(4-bromo-2-fluorophenyl)ethylidene)-2-methylpropane-2-sulfinamide (C4)

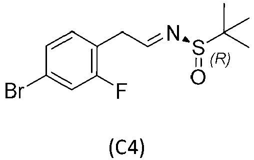

**Reaction:** To a reaction kettle were added a solution of C3 (37.0 kg, 1.0 eq) in toluene, (S)-tert-butylsulfinamide (1.3 eq) and anhydrous copper sulfate (3.0 eq) under stirring. After the addition was completed, under nitrogen atmosphere, the reaction solution was warmed to 40°C ± 5°C and stirred for 10 h with the temperature maintained constant. The reaction mixture was sampled and monitored. When the in-process control limit of C3/C3 + C4 **≤** 20.0% was met, the reaction was stopped.

**Workup:** After the reaction was completed, the reaction solution was filtered over diatomaceous earth. The filter cake was rinsed with toluene (1V), and the filtrate was collected. The filtrate was washed twice with a 5% sodium bicarbonate solution and allowed to stand for phase separation, to obtain a solution of C4 in toluene (total weight: 859.5 kg, content: 4.6%, yield: 72.4%), which was directly used in the next reaction.

¹H NMR (300 MHz, CDCl₃) δ 8.10 - 7.96 (m, 1H), 7.18 (m, 2H), 7.09 - 6.92 (m, 1H), 3.85 - 3.66 (m, 1H), 1.08 (s ,9H).

LCMS m/z =320.0 [M+1]⁺
The C4 in this step can also be prepared using the following method:
**Reaction:** To a reaction kettle were added a solution of C3 (27.0 kg, 1.0 eq) in toluene (370 kg), (S)-tert-butylsulfinamide **(1.3** eq.), acetic acid (2.5 V) and molecular sieve (108 kg) under stirring. After the addition was completed, the mixture was purged three times with nitrogen, warmed to 40°C ± 5°C, and stirred for 12 hours with the temperature maintained constant. The reaction mixture was sampled and monitored. When the in-process control limit of C3/C3 + C4 **≤** 20.0% was met, the reaction was stopped.

**Workup:** After the reaction was completed, the reaction solution was cooled to 20°C ± 5°C and filtered over diatomaceous earth. The filter cake was rinsed with toluene (2V), and the filtrate was collected. The filtrate was washed twice with a 5% sodium bicarbonate solution and allowed to stand for phase separation, to obtain a solution of C4 in toluene (total weight: 366.6 kg, content: 5.0%, yield: 67.2%), which was directly used in the next reaction.

### Step 4: (R)-N-((S)-2-(4-bromo-2-fluorophenyl)-1-cyanoethyl)-2-methylpropane-2-sulfinamide (C5-1)

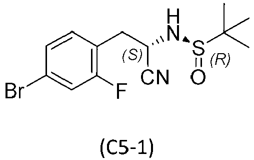

**Reaction:** To a reaction kettle was added a solution of C4 (39.5 kg, 1.0 eq) in toluene, and stirring was then initiated, followed by the addition of cesium fluoride (0.2 eq). The mixture was purged three times with nitrogen and cooled to 0°C ± 5°C under nitrogen atmosphere. Trimethylsilyl cyanide (1.5 eq) was slowly added dropwise with the temperature controlled at 0°C ± 5°C during the dropwise addition. After the dropwise addition was completed, the mixture was stirred for 12 hours with the temperature maintained at 0°C ± 5°C. The reaction mixture was sampled and monitored. When the in-process control limit of C4/C4 + C5 **≤** 2.0% was met, the reaction was stopped.

**Workup:** After the reaction was completed, a 1% sodium carbonate solution was added to the system to quench the reaction, and the pH was adjusted to approximately 8. The system was allowed to stand for phase separation to obtain the organic phase. The organic phase was successively washed with a mixed solution of 0.5% sodium carbonate and 3.0% sodium chloride (5V×3) and a 5.0% sodium chloride solution (5V×2). Then, the resulting system was allowed to stand for phase separation, and the organic phase was collected, to obtain a solution of C5-1 in toluene (total weight: 862.5 kg, content: 4.3%, yield: 88.2%), which was directly used in the next reaction.

LCMS m/z =347.2[M+1]⁺
The C5-1 in this step can also be prepared using the following method:
**Reaction:** To a reaction kettle was added a solution of C4 (0.2 kg, 1.0 eq) in toluene (2 kg, 1.0 eq), and stirring was then initiated, followed by the addition of potassium fluoride (0.2 eq). The mixture was cooled to 0°C ± 5°C under nitrogen atmosphere, and trimethylsilyl cyanide (1.5 eq) was slowly added dropwise. After the dropwise addition was completed, the mixture was stirred for 16 hours with the temperature controlled at 0°C ± 5°C. The reaction mixture was sampled and monitored. When the in-process control limit of C4/C4 + C5 **≤** 2.0% was met, the reaction was stopped.

**Workup:** After the reaction was completed, a 5% sodium carbonate solution was added to the system to quench the reaction, and the pH was adjusted to approximately 8. The system was allowed to stand for phase separation to obtain the organic phase. The organic phase was successively washed with a mixed solution of 5.0% sodium carbonate solution and 5.0% sodium chloride solution. Then, the resulting system was allowed to stand for phase separation, and the organic phase was collected and concentrated to dryness to obtain a solution of C5-1 in toluene (crude: 0.23 kg, yield: 100%), which was directly used in the next reaction.

### Step 5: (S)-2-amino-3-(4-bromo-2-fluorophenyl)propanenitrile hydrochloride (C6)

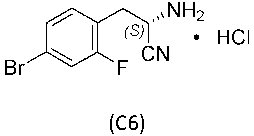

**Reaction:** To a reaction kettle was added a solution of C5-1 (37.0 kg, 1.0 eq) in toluene, and stirring was then initiated. The mixture was purged three times with nitrogen and cooled to 0°C ± 5°C under nitrogen atmosphere. Hydrochloric acid/ethanol (2.5 eq) was slowly added dropwise with the temperature controlled at 0°C ± 5°C during the dropwise addition. After the dropwise addition was completed, the mixture was warmed to 20°C ± 5°C and stirred for 4 hours with the temperature maintained constant. The reaction mixture was sampled and monitored. When the in-process control limit of C5-1/C6 + C5-1 **≤** 2.0% was met, the reaction was stopped.

**Workup:** After the reaction was completed, the reaction mixture was cooled to 10°C ± 5°C, stirred for 1 hour, and filtered. The filter cake was washed with MTBE (2V), and the filter cake was collected to obtain a wet product of C6 (total weight: 63.8 kg, content: 63.8%, yield: 100%), which was directly used in the next reaction.

¹H NMR (400 MHz, DMSO) δ 9.00 (s, 2H), 7.64 (dd, 1H), 7.45 - 7.37 (m, 2H), 4.84 (t, 1H), 3.26 - 3.16 (m, 2H).

LCMS m/z =243.0[M-HCl+H]⁺

### Step 6: (S)-2-amino-3-(4-bromo-2-fluorophenyl)propanenitrile L-tartrate (C6-b)

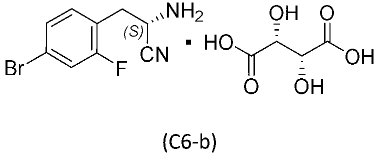

**Reaction:** To a reaction kettle were added C6 (58.4 kg, 1.0 eq) and dichloromethane (6V), and stirring was then initiated. The mixture was purged three times with nitrogen and cooled to 5°C ± 5°C, and a 5% sodium carbonate solution (10V) was added dropwise with the temperature controlled at 5 ± 5°C. After the dropwise addition was completed, the mixture was warmed to 20°C ± 5°C and stirred for 1 hour with the temperature maintained constant. The system was allowed to stand for phase separation, and the aqueous phase was extracted once with DCM (3V). The organic phases were combined and concentrated to about half of the initial volume. The resulting system was cooled to 20°C ± 5°C, and MeOH (3.5V) was added, followed by the dropwise addition of a solution of L(-)-tartaric acid (0.8 eq) in methanol (3.5V). After the dropwise addition was completed, the mixture was stirred for 4 hours with the temperature maintained at 15°C ± 5°C.

**Workup:** After the reaction was completed, the mixture was filtered, and the filter cake was washed with MTBE (3V). The filter cake was collected to obtain a wet product of C6-b (total weight: 92.2 kg, content (free base): 36.9%, yield: 67.0%), which was directly used in the next reaction.
Purity: 98.2%
Chiral purity: 99.6%

### Step 7: tert-butyl (S)-(2-(4-bromo-2-fluorophenyl)-1-cyanoethyl)carbamate (C7)

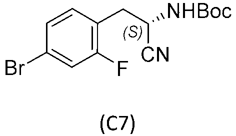

**Reaction:** To a reaction kettle were added C6-b (54.8 kg, 1.0 eq) and dichloromethane MTBE (6V), and stirring was then initiated. The mixture was cooled to 0°C ± 5°C, and a 5% sodium carbonate solution (1.1 eq) was added dropwise. After the dropwise addition was completed, the mixture was stirred for 20 min with the temperature controlled at 0°C ± 5°C, and a solution of (Boc)₂O (1.6 eq) in MTBE (1V) was added dropwise to the system. After the dropwise addition was completed, the mixture was warmed to 20°C ± 5°C and stirred for 15 hours with the temperature maintained constant. The reaction mixture was sampled and monitored. When the in-process control limit of C6-b/C6-b + 7 **≤** 1.0% was met, the reaction was stopped.

**Workup:** After the reaction was completed, the system was allowed to stand for phase separation. The aqueous phase was extracted once with MTBE (1V), and the organic phases were combined. n-Heptane (10V) was added, and then the mixture was stirred and heated to 35°C ± 5°C, and concentrated to about one-tenth of the initial volume. The concentrate was cooled to 10°C ± 5°C, and n-heptane (10V) was added. The mixture was stirred for 3 h and then filtered. The filter cake was washed with a mixture of MTBE : n-heptane = 1 : 7 (2V) to obtain a wet product of C7. The wet product was dried at 35°C ± 5°C for about 16 hours, to obtain compound C7 (weight: 34.4 kg, yield: 71.0%).
Purity: 99.6%
Chiral purity: 100.0%
¹H NMR (400 MHz, DMSO) δ 7.84 (s, 1H), 7.54 (dd, 1H), 7.47 - 7.28 (m, 2H), 4.69 (d, 1H), 3.08 (qd, 2H), 1.38 (s, 9H).
LCMS m/z =287.0[M-56+H]⁺.

In summary, the method for preparing the compound as represented by formula (I) provided by the present disclosure has advantages such as low-cost reaction starting materials, multi-step telescoping reactions, straightforward workup procedures, high yield, high chemical and chiral purity of the product, and the suitability for large-scale industrial production.

## Claims

1. A method for preparing a compound as represented by formula (I), comprising 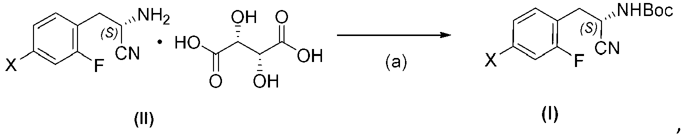
wherein X is a leaving group, such as F, Cl, Br, I or OTf;
a: the compound as represented by formula (I) is prepared from a compound of formula (II).

2. The preparation method according to claim 1, wherein the method further comprises the following steps: 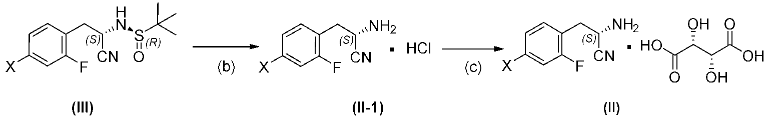
wherein X is a leaving group, such as F, Cl, Br, I or OTf;
b: a compound of formula (II-1) is prepared from a compound of formula (III);
c: the compound of formula (II) is prepared from the compound of formula (II-1).

3. A method for preparing a compound as represented by formula (II), comprising 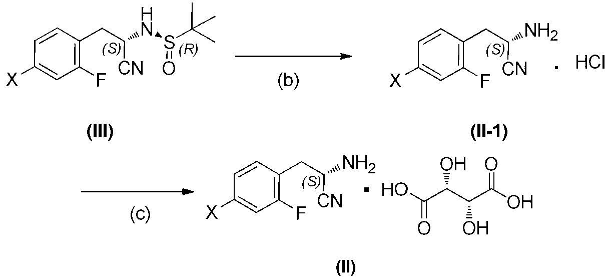
wherein X is a leaving group, such as F, Cl, Br, I or OTf;
b: a compound of formula (II-1) is prepared from a compound of formula (III);
c: the compound of formula (II) is prepared from the compound of formula (II-1).

4. The preparation method according to claim 2 or 3, wherein the method further comprises the following steps: 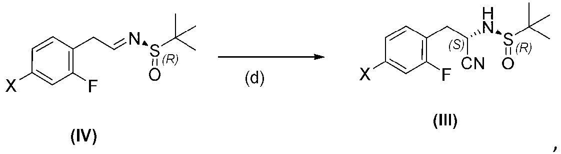
wherein X is a leaving group, such as F, Cl, Br, I or OTf;
d: the compound of formula (III) is prepared from a compound of formula (IV).

5. A method for preparing a compound as represented by formula (III), comprising 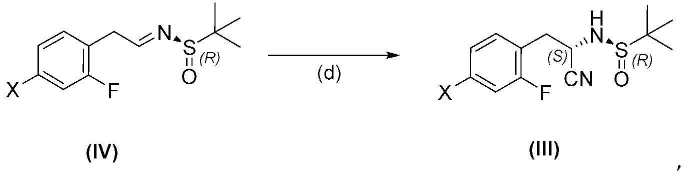
wherein X is a leaving group, such as F, Cl, Br, I or OTf;
d: the compound of formula (III) is prepared from a compound of formula (IV).

6. The preparation method according to claim 4 or 5, wherein the method further comprises the following steps: 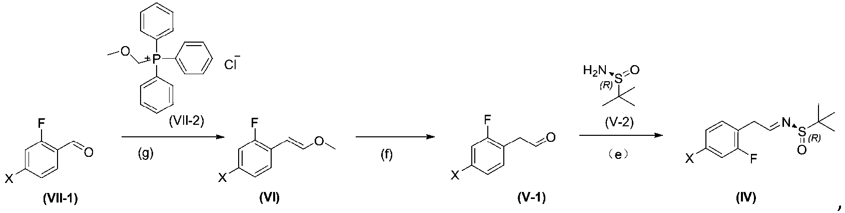
wherein X is a leaving group, such as F, Cl, Br, I or OTf;
g: a compound of formula (VI) is prepared from a compound of formula (VII-1) and a compound of formula (VII-2);
f: a compound of formula (V-1) is prepared from the compound of formula (VI);
e: the compound of formula (IV) is prepared from the compound of formula (V-1) and a compound of formula (V-2).

7. A method for preparing a compound as represented by formula (IV), comprising 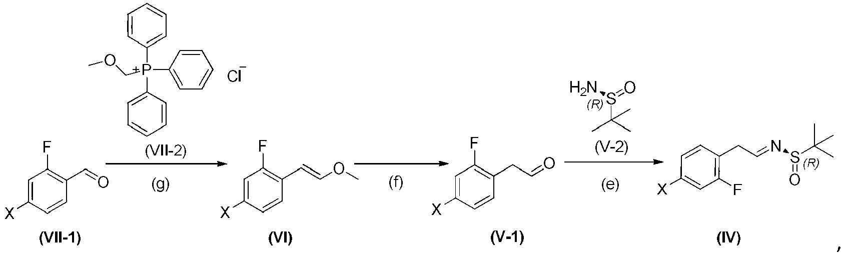
wherein X is a leaving group, such as F, Cl, Br, I or OTf;
g: a compound of formula (VI) is prepared from a compound of formula (VII-1) and a compound of formula (VII-2);
f: a compound of formula (V-1) is prepared from the compound of formula (VI);
e: the compound of formula (IV) is prepared from the compound of formula (V-1) and a compound of formula (V-2).

8. The preparation method according to any one of claims 1, 2, 4 and 6, wherein
a: the compound as represented by formula (II) is reacted in the presence of an organic solvent and a base to obtain the compound as represented by formula (I),
wherein the organic solvent is preferably one or more of dichloromethane, ethyl acetate, isopropyl acetate, toluene, dioxane, tetrahydrofuran, methyltetrahydrofuran, MTBE, (Boc)₂O or n-heptane, more preferably one or more of dichloromethane, MTBE, (Boc)₂O or n-heptane; the base is preferably one or more of sodium carbonate, sodium bicarbonate, potassium carbonate, potassium bicarbonate, potassium phosphate, potassium hydroxide, lithium hydroxide, sodium hydroxide, triethylamine, DIPEA or DBU, more preferably sodium carbonate.

9. The preparation method according to any one of claims 2, 3, 4 and 6, wherein
b: the compound of formula (III) is reacted in the presence of an organic solvent and an acid to obtain the compound of formula (II-1),
wherein the organic solvent is preferably one or more of dichloromethane, ethyl acetate, isopropyl acetate, toluene, dioxane, tetrahydrofuran, methyltetrahydrofuran, MTBE, methanol, ethanol or isopropanol, more preferably ethanol or MTBE;
the acid is preferably one or more of HCl, hydrobromic acid, hydroiodic acid, sulfuric acid, phosphoric acid, methanesulfonic acid, p-toluenesulfonic acid or trifluoroacetic acid, more preferably HCl;
c: the compound of formula (II-1) is reacted in the presence of a chiral acid solvent to obtain the compound of formula (II),
wherein the chiral acid solvent is preferably one or more of tartaric acid, malic acid, camphoric acid, camphorsulfonic acid, lactic acid, diacetone-L-gulonic acid, mandelic acid or phenoxypropionic acid, more preferably L(-)-tartaric acid.

10. The preparation method according to any one of claims 4-6, wherein
d: the compound of formula (IV) is reacted in the presence of a solvent, a cyanating reagent and a base to obtain the compound of formula (III),
wherein the solvent is preferably one or more of DCM, DMF, THF, n-heptane, ACN, toluene or DMSO, more preferably DCM or toluene;
the cyanating reagent is preferably one or more of TMSCN, sodium cyanide, potassium cyanide or lithium cyanide, more preferably TMSCN;
the base is preferably one or more of CsF, KF, TBAF, Na₂CO₃, K₃PO₄ or Ti(OEt)₄, more preferably CsF or KF, even more preferably CsF.

11. The preparation method according to claim 6 or 7, wherein
g: the compound of formula (VII-1) and the compound of formula (VII-2) are reacted in the presence of a base to obtain the compound of formula (VI),
wherein the base is preferably one or more of LiHMDS, NaHMDS, KHMDS, LDA, BuLi or potassium tert-butoxide, more preferably LiHMDS, NaHMDS or KHMDS, even more preferably LiHMDS;
f: the compound of formula (VI) is reacted in the presence of an acid and an organic solvent to obtain the compound of formula (V-1),
wherein the acid is preferably one or more of TsOH, methanesulfonic acid, camphorsulfonic acid, hydrochloric acid, hydrobromic acid, hydroiodic acid, sulfuric acid, phosphoric acid, trifluoroacetic acid, oxalyl chloride or trimethylchlorosilane, more preferably TsOH;
the organic solvent is preferably one or more of dichloromethane, ethyl acetate, isopropyl acetate, toluene, dioxane, tetrahydrofuran, methyltetrahydrofuran, methyl tert-butyl ether, acetonitrile, methanol, ethanol or isopropanol, more preferably toluene;
e: the compound of formula (V-1) and the compound of formula (V-2) are reacted in the presence of an organic solvent and a dehydration reagent to obtain the compound of formula (IV),
wherein the organic solvent is preferably one or more of toluene, dichloromethane, ethyl acetate, isopropyl acetate, dioxane, tetrahydrofuran, methyltetrahydrofuran, acetic acid or acetonitrile, more preferably toluene;
the dehydration reagent is preferably one or more of anhydrous CuSO₄, Zn(OAc)₂, Co(OAc)₂^{·}4H₂O, Na₂SO₄, MgSO₄, Ti(Oi-Pr)₄, DCC/DMAP, EDCI/DMAP or HOAc/molecular sieve, more preferably anhydrous CuSO₄ or HOAc/molecular sieve.

12. A method for preparing a compound as represented by formula (C7), comprising 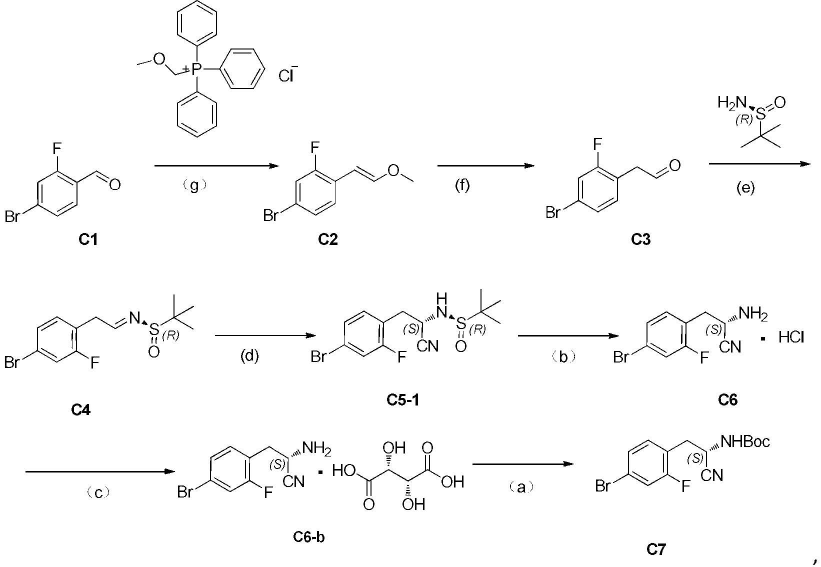 wherein
g: C1 is reacted in the presence of an organic solvent and a base to obtain C2;
f: C2 is reacted in the presence of an organic solvent and an acid to obtain C3;
e: C3 is reacted in the presence of an organic solvent and a dehydration reagent to obtain C4;
d: C4 is reacted in the presence of a solvent, a base and a cyanating reagent to obtain C5-1;
b: C5-1 is reacted in the presence of an acid to obtain C6;
c: C6 is reacted in the presence of an organic solvent, a base and a chiral acid to obtain C6-b;
a: C6-b is reacted in the presence of an organic solvent and a base to obtain C7.

13. The preparation method according to claim 12, wherein
g: the organic solvent is selected from one or more of tetrahydrofuran, dichloromethane, MTBE or n-heptane;
the base is selected from one or more of LiHMDS, NaHMDS or KHMDS, preferably LiHMDS;
f: the acid is selected from one or more of TsOH, methanesulfonic acid, camphorsulfonic acid, hydrochloric acid, hydrobromic acid, hydroiodic acid, sulfuric acid, phosphoric acid, trifluoroacetic acid, oxalyl chloride or trimethylchlorosilane, preferably TsOH;
the organic solvent is selected from one or more of dichloromethane, ethyl acetate, isopropyl acetate, toluene, dioxane, tetrahydrofuran, methyltetrahydrofuran, methyl tert-butyl ether, acetonitrile, methanol, ethanol or isopropanol, preferably toluene;
e: the organic solvent is selected from one or more of toluene, dichloromethane, ethyl acetate, isopropyl acetate, dioxane, tetrahydrofuran, methyltetrahydrofuran, acetic acid or acetonitrile, preferably toluene; and the dehydration reagent is selected from one or more of anhydrous CuSO₄, Zn(OAc)₂, Co(OAc)₂^{·}4H₂O, Na₂SO₄, MgSO₄, Ti(Oi-Pr)₄, DCC/DMAP, EDCI/DMAP or HOAc/molecular sieve, preferably anhydrous CuSO₄ or HOAc/molecular sieve;
d: the solvent is selected from one or more of DCM, DMF, THF, n-heptane, ACN, toluene or DMSO, preferably DCM or toluene; the cyanating reagent is selected from one or more of TMSCN, sodium cyanide, potassium cyanide or lithium cyanide, preferably TMSCN; and the base is selected from one or more of CsF, KF, TBAF, Na₂CO₃, K₃PO₄ or Ti(OEt)₄, preferably CsF or KF, even more preferably CsF;
b: the organic solvent is selected from one or more of dichloromethane, ethyl acetate, isopropyl acetate, toluene, dioxane, tetrahydrofuran, methyltetrahydrofuran, MTBE, methanol, ethanol or isopropanol, preferably ethanol or MTBE; and the acid is selected from one or more of HCl, hydrobromic acid, hydroiodic acid, sulfuric acid, phosphoric acid, methanesulfonic acid, p-toluenesulfonic acid or trifluoroacetic acid, preferably HCl;
c: the chiral acid solvent is selected from one or more of tartaric acid, malic acid, camphoric acid, camphorsulfonic acid, lactic acid, diacetone-L-gulonic acid, mandelic acid or phenoxypropionic acid, preferably L(-)-tartaric acid;
a: the organic solvent is selected from one or more of dichloromethane, ethyl acetate, isopropyl acetate, toluene, dioxane, tetrahydrofuran, methyltetrahydrofuran, MTBE, (Boc)₂O or n-heptane, more preferably one or more of dichloromethane, MTBE, (Boc)₂O or n-heptane; and the base is selected from one or more of sodium carbonate, sodium bicarbonate, potassium carbonate, potassium bicarbonate, potassium phosphate, potassium hydroxide, lithium hydroxide, sodium hydroxide, triethylamine, DIPEA or DBU, preferably sodium carbonate.

14. The preparation method according to claim 12 or 13, wherein
g: the organic solvent is tetrahydrofuran, and the base is LiHMDS;
f: the acid is TsOH, and the organic solvent is toluene;
e: the dehydration reagent is selected from anhydrous CuSO₄ or HOAc/molecular sieve;
d: the cyanating reagent is TMSCN, and the base is CsF;
b: the organic solvent is selected from ethanol or MTBE, and the acid is HCl;
c: the chiral acid solvent is L(-)-tartaric acid;
a: the organic solvent is selected from one or more of dichloromethane, MTBE, (Boc)₂O or n-heptane; and the base is sodium carbonate.
